# EUROPEAN PATENT SPECIFICATION

(11) **EP 2 512 548 B1**
(45) Date of publication and mention of the grant of the patent: **06.12.2017**
(21) Application number: 10812887.7
(22) Date of filing: 14.12.2010
(51) Int. Cl.: A61M 1/34, A61M 5/14, A61M 39/28

(54) **HOLDING ELEMENT FOR FLEXIBLE TUBE CLAMPING DEVICES IN MEDICAL MACHINES**
HALTEELEMENT FÜR SCHLAUCHKLEMMEN BEI MEDIZINISCHEN MASCHINEN
ÉLÉMENT DE MAINTIEN POUR DISPOSITIFS DE SERRAGE DE TUBES SOUPLES DANS DES MACHINES MÉDICALES

(30) Priority: 15.12.2009 IT MI20092190
(43) Date of publication of application: 24.10.2012
(73) Proprietor: Gambro Lundia AB, 220 10 Lund (SE)
(72) Inventor: PARALUPPI, Marco, 41036 Medolla (IT); LITTERA, Michele, 41037 Mirandola (IT)
(74) Representative: Ponzellini, Gianmarco
(86) International application number: PCT/IB2010/003239
(87) International publication number: WO 2011/073766

(56) References cited:
- EP-A1- 2 253 350
- US-A1- 2003 040 700
- US-A1- 2008 287 854
- US-A1- 2008 294 122

## Description

### Background of the Invention

The invention relates to a holding element for flexible hose clamping devices in medical machines.

Specifically, though not exclusively, the invention can be used in the medical field to maintain flexible hoses in position in devices for regulating extracorporeal flow of medical liquids and/or biological fluids.

In particular, the device in object is applicable in an hemo(dia)filtration apparatus to enable insertion, maintaining in position and extraction of a flexible hose for fluid transport in a device for clamping the tubes, for example of the type destined to selectively enable passage of blood in removal lines and/or return lines of blood to a patient. The device can also be of the type destined to selectively enable distribution of a replacement fluid in the pre-dilution lines and post-dilution lines of a medical machine.

The holding element and the corresponding device can further find application in further applicational fields that are not described in detail in the present description.

In the prior art, various devices are known for clamping hoses for fluid transport, in which the devices enable selective interruption of or in any case regulation of the passage of fluids in hoses inserted in the devices. These clamping devices in general comprise a housing seating for a tract of tubing and a piston that is selectively mobile for enabling fluid passage in the tract of hose, when the piston is in retracted position with respect to the hose, or for regulating or selectively interrupting the fluid passage by squeezing the tract of hose by means of a controlled advancement of the piston.

The known-type clamping devices are further provided with holding elements which are selectively mobile between an operating position in which they enable insertion and/or extraction of a tract of hose from the housing seating of the clamping device and an operating position in which they prevent extraction of the tract of tubing from the device, thus maintaining the tract of hose in position during the functioning of the medical machine and preventing the hose from being inadvertently separated from the clamping device, with consequent considerable risks for the patient subjected to treatment.

The holding elements of known type generally comprise a selectively mobile part which can be displaced between a retracted position internally of the frame of the medical machine or the clamping device and an advanced position, extracted from the frame of the medical machine or the clamping device and destined to prevent extraction of the tract of hose from the clamping device.

This type of holding element and corresponding clamping devices exhibit some drawbacks. The holding elements and the device of know type are difficult to clean as they comprise mobile parts which in the retracted position go inside a frame. Any undesired and dirtying substances can in fact be drawn internally of the machine frame of the device in the retracted position, in zones which are difficult to access for cleaning Further, the holding elements and known-type devices are difficult to demount both in order to clean the various parts, and to enable replacement of any damaged or contaminated parts.

The above-cited problems are particularly relevant in the case of medical machines and in particular in the case of use with hoses for transport of corporeal fluids, typically blood, as in a case of contamination of the holding element or the clamping device with the corporeal fluids, there is the risk that a difficult or insufficient cleaning of the involved parts might lead to serious risks to the persons using the machine or other patients.

To obviate in part these drawbacks, holding elements have been provided which do not enter internally of the frame of the machine or the clamping device in the relative disengaged position thereof. Even these devices do not however obviate the above-indicated problems, as they do not enable an effective cleaning of the clamping device which all the same exhibits zones that are difficult to access due to the presence of the holding element or due to the conformation of the device for clamping or the holding device, which exhibit various concave parts or holes that are difficult to access.

Also known from document US2003 0040700 is a holding element for engaging flexible hoses in which a main body exhibits two hook-shaped opposite engaging portions for removably engaging a machine body in infusion devices.

The holding element retains the flexible hose, positioning it such that a pump can be active on a tract of the hose for moving the fluid contained internally thereof.

### Summary of the Invention

An aim of the present invention is to provide a holding element, for flexible hose clamping devices in medical machines, and a corresponding clamping device, which enable easy and rapid cleaning thereof. A further aim of the present invention is to provide a holding element, for flexible hose clamping devices in medical machines, and a corresponding clamping device which offer a high safety level against any contaminations from liquids flowing through the flexible hoses.

A further aim of the present invention is to provide a holding element, for flexible hose clamping devices in medical machines, and a corresponding clamping device which are simple, economical, reliable, of compact dimensions and of easy and immediate use.

At least one of the above-indicated aims is attained by a holding element for flexible hose clamping devices in medical machines and for a corresponding clamping according to the claims. In a further aspect, the invention relates to a holding element in which a portion of mounting is at least partially complementarily-shaped to at least a head portion of a clamping device.

In a further aspect, the invention relates to a holding element in which the mounting portion comprises at least two rest surfaces which are opposite and destined to enter into contact, when the holding element is mounted on a clamping device, with corresponding opposite surfaces of the corresponding head portion of the claiming device, in order to maintain the holding element in position on the clamping device.

In a further aspect, the invention relates to a holding element in which the mounting portion comprises four rest surfaces which are transversal to one another and reciprocally connected and destined to enter into contact, when the holding element is mounted on a clamping device, with corresponding surfaces of the corresponding head portion of the clamping device that are connected to one another, in order to maintain the holding element on the clamping device.

In a further aspect, the invention relates to a holding element in which the mounting portion is conformed in such a way as to maintain the holding element stably mounted on the device in the engaged position and in the access position.

In a further aspect, the invention relates to a holding element conformed in such a way that a pushing action destined to bring the retaining portion into an access position for a hose involves a pushing of the mounting portion towards the clamping device.

In a further aspect, the invention relates to a holding element in which the mounting exhibits an internal surface, couplable with the device, without projections insertable in the device.

In a further aspect, the invention relates to a holding element in which the support portion is entirely elastically deformable.

In a further aspect, the invention relates to a holding element in which the zone of the support portion connected to the mounting portion is defined by through-cuts realised in the mounting portion.

In a further aspect, the invention relates to a holding element in which the body is realised, in several parts which are reciprocally mounted.

In a further aspect, the invention relates to a holding element in which the elastically deformable zone is defined in the mounting portion.

In a further aspect, the invention relates to a holding element in which the elastically deformable zone is defined by at least a structurally-weakened line in the mounting portion or the support portion.

In a further aspect, the invention relates to a holding element in which the elastically deformable zone is defined by means of at least a cut in the mounting portion or the support portion, defining a weakened zone destined to facilitate bending of the support portion.

In a further aspect, the invention relates to a holding element of the single-use variety.

In a further aspect, the invention relates to a holding element in which the retaining portion comprises an activating portion destined to enable and facilitate a manual displacement of the holding portion.

In a further aspect, the invention relates to a holding element in which the body is realised in a plastic material.

In a further aspect, the invention relates to a clamping device which further comprises a head portion to which the mounting portion of the holding element is removably mountable, the head portion of the device having an external surface, couplable with the mounting portion, which is continuous and without openings.

In a further aspect, the invention relates to a hemo(dia)filtration apparatus comprising: a hemo(dia)filter having a blood chamber and a fluid chamber which are separated from one another by a semipermeable membrane; an extracorporeal blood circuit having a blood removal line which is connected to an inlet of the blood chamber and a blood return line which is connected to an outlet of the blood chamber; and at least a device for clamping flexible hoses realised according to any one of the accompanying device claims, and operatively associated to the removal line or the return line, and at least a holding element according to any one of the accompanying holding element claims.

In a further aspect, the invention relates to a hemo(dia)filtration apparatus further comprising a replacement fluid circuit having a pre-dilution line connected to the removal line and a post-dilution line connected to the return line; and at least a device for clamping flexible hoses which is realised according to any one of the accompanying device claims, and operatively associated to the removal line or the return line, or the pre-dilution line or the post-dilution line, and at least a holding element according to any one of the accompanying holding element claims. Further characteristics and advantages of the present invention will better emerge from the detailed description that follows of at least an embodiment of the invention, illustrated by way of non-limiting example in the accompanying figures of the drawings.

### Brief Description of the Drawings

The detailed description will be made herein below with reference to the accompanying figures of the drawings, provided by way of non-limiting example, and in which:
- figure 1 is a perspective view of two devices for clamping flexible hoses and two holding elements mounted on the devices, in agreement with an embodiment of the present invention, in an engaged working position;
- figure 2 is a second perspective view of the devices and the holding elements of figure 1;
- figure 3 is a perspective view of one of the devices for clamping and one of the holding elements of figure 1 in an operative access position;
- figure 4 is a lateral view of the holding device and element of figure 3 in the operative access position;
- figure 5 is a perspective view of the holding devices and elements of figure 1, in the operative holding position and with respective hoses housed in the devices themselves;
- figure 6 is a further perspective view of the devices of figure 5 in which one of the holding elements has been removed by a respective device;
- figure 7 is a lateral view of the holding devices and elements of figure 6.

### Detailed Description

With reference to the figures of the drawings, 1 denotes in its entirety a device for clamping flexible hoses 2. By "clamping", in the present description we refer to the act of squeezing a tract of flexible tubing in order to regulate or completely interrupt a fluid flow passing internally of the flexible hose.

The clamping device 1 comprises a housing seating 3 for a flexible hose 2 and a pinch for flexible hoses 2 having a piston that is mobile in an axial direction. The piston solidly bears a crushing projecting element 4 which operates in collaboration with a fixed abutting device 5 for occluding by squeezing a first tract of flexible hose 2 housed in the housing seating 3.

In operating conditions, the piston emerges from the frame of the medical machine to which the device is associated, and moves in an external direction such as to reach the fixed abutting device 5 (when the hose is interposed the piston squeezes it against the fixed abutment and causes clamping).

The pinch can assume at least an open configuration, in which it defines a housing seating 3 for receiving a tract of flexible hose 2, and at least a closed configuration, in which it closes by crushing the tract of flexible hose 2 receiving the housing seating 3. The clamping device 1 can comprise an elastic element for displacing the pinch towards a respective open or closed configuration.

The elastic element can comprise a spring coupled to the piston. The clamping device 1 can further comprise an activating device for displacing the pinch towards the respective open and closed configurations. The clamping device 1 can further comprise at least a position sensor configured for issuing a position signal indicating at least a position of the piston. The clamping device 1 can further comprise a control unit which receives the position signal and which controls the position of the piston on the basis of the position signal.

The clamping device 1 can further comprise a presence sensor 6 of the flexible hose 2, internally of the housing seating 6, which presence sensor 6 is interfaced with the control unit.

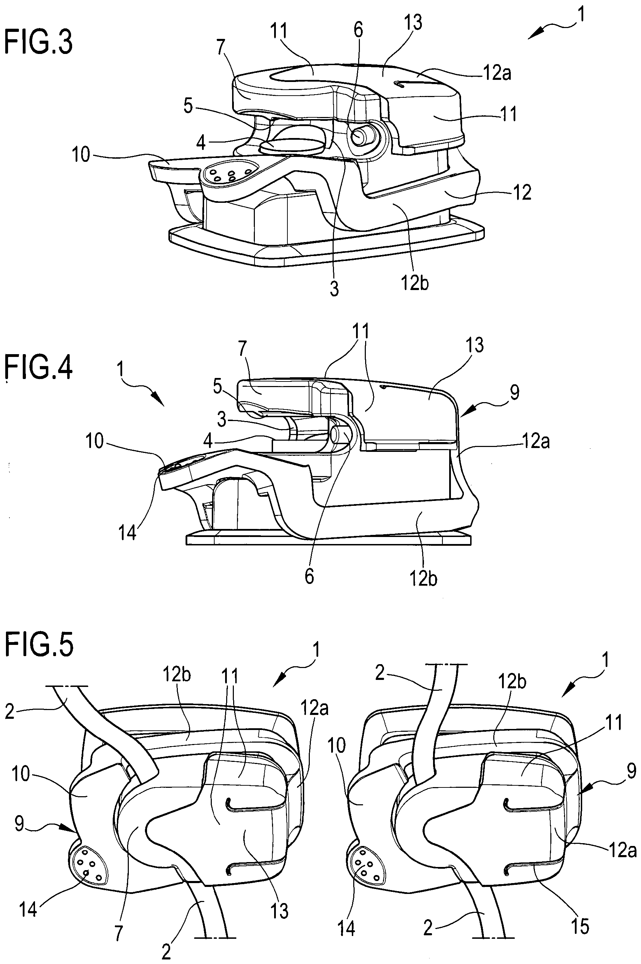

In section, the housing seating 3 exhibits a C-profile and has an elongate shape along a development direction that coincides with the flexible hose to be received (figure 4).

The retaining portion 10 of the holding element 9 described herein below will be positioned, with respect to the seating 3, in engaged conditions of the holding element to the head 7, such as to prevent an extraction of the flexible hose 2 from the seating 3, blocking the outlet hole of the seating 3; a movement of the retaining portion 10 towards the access position frees the outlet hole of the seating 3 and enables the flexible hose 2 to be removed.

The above-cited elements of the clamping device 1 are in themselves substantially of known type and for this reason are not illustrated and described in greater detail in the present description. In the device, the above-mentioned head portion 7 is provided with a recessed seating 8 which will be described in greater detail herein below.

The seating 3 is in fact entirely realised in the head portion 7 which exhibits the seating for sliding the piston and which superiorly also defines the abutting device 5.

Looking at figure 3, it can be seen that the piston is mobile in the seating 3 below the upper surface of the head portion 7 which incorporates the clamping system of the hose (seating 3, piston 4 and abutting device 5).

In the present invention, the device 1 further comprises at least a holding element 9 which is removably mountable to a head portion 7 and has a retaining portion 10 destined, in the holding position, to maintain the tract of flexible hose 2 in the housing seating 3, and destined, in the access position, to enable insertion of the tract of flexible hose 2 into the housing seating 3 or extraction of the tract of flexible hose 2 from the housing seating 3.

In the invention, the holding element 9 for clamping devices of flexible hoses in medical machines, is provided with at least a body comprising at least a mounting portion 11 that is removably mountable to a clamping device 1 for flexible hoses 2. The body further comprises the retaining portion 10 of a flexible hose 2 and at least a support portion 12, interposed between the mounting portion 11 and the support portion 12, interposed between the mounting portion 11 and the sealing portion 10.

The body is provided with at least an elastically deformable zone 13 for enabling selective displacement of the retaining portion 10 with respect to the mounting portion 11 when the holding element 9 is mounted on a corresponding clamping device 1 for flexible hoses 2, between an engaged position in which it is destined to maintain a tract of flexible hose 2 in position with respect to the device 1 and at least an access position, displaced with respect to the engaged position, in which it is destined to enable insertion and/or extraction of a tract of flexible hose 2 into or from the device 1. In the illustrated embodiment the elastically deformable zone 13 is defined in the support portion 12.

The elastically deformable zone 13 can be defined by through-recesses 15 realised in the mounting portion 11 and defining a weakened zone destined to facilitate the bending of the support portion 12. In particular the support portion 12 comprises a single first arm 12a extending from the first arm and a pair of secondary arms 12b extending extending from the first arm up to two ends of the holding portion. As can be seen in the figures, the zone of the support portion 12 connected to the mounting portion 11 is defined by through-recesses 15 realised in the mounting portion 11. In a variant embodiment, the body can comprise two support portions 12 extending from opposite sides for supporting opposite sides of the retaining portion 10.

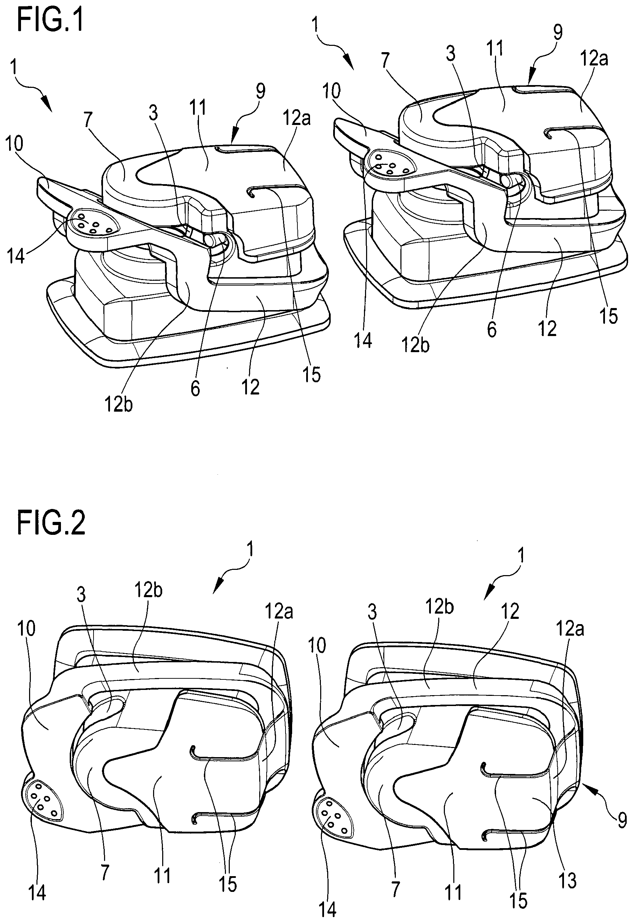

In the illustrated embodiment, at least the part of the support portion 12 constituted by the single first arm 12a is elastically deformable. The retaining portion 10 is conformed such as to be in the holding position (illustrated for example in figures 1, 2 and 5) with the support portion 12 at rest, and to be displaceable into the access position (illustrated for example in figures 3 and 4) by a pushing action, in a first direction (or more precisely in a first direction along an activating direction) destined to overcome an elastic resistance of the support portion 12.

As can be seen in figure 5, in this way the holding element 9 enables the tract of hose 2 to be maintained housed in the device 1 when at rest, and to be activated by exerting a pushing action in the first direction in order to displace the retaining portion 10 into the access position in which it enables extraction of the tract of hose 2 from the device 1. The mounting portion 11 can be freed from the device by means of a pushing action on the retaining portion 10 in a second direction, substantially opposite the first direction (or more precisely in a second sense along the same activating direction or along a parallel direction). The mounting portion 11 is at least partially complementarily shaped to at least a head portion 7 of the clamping device 1.

The head portion 7 of the device is provided with at least a recessed housing 8 positioned superiorly of the housing seating 3 and at the fixed abutting device 5 of the seating and complementarily-shaped with respect to at least a part of the mounting portion 11 of the holding element 9 in such a way that the mounting portion 11 is stably insertable in the recessed housing 8 in order to prevent accidental extraction of the holding element 9 from the device.

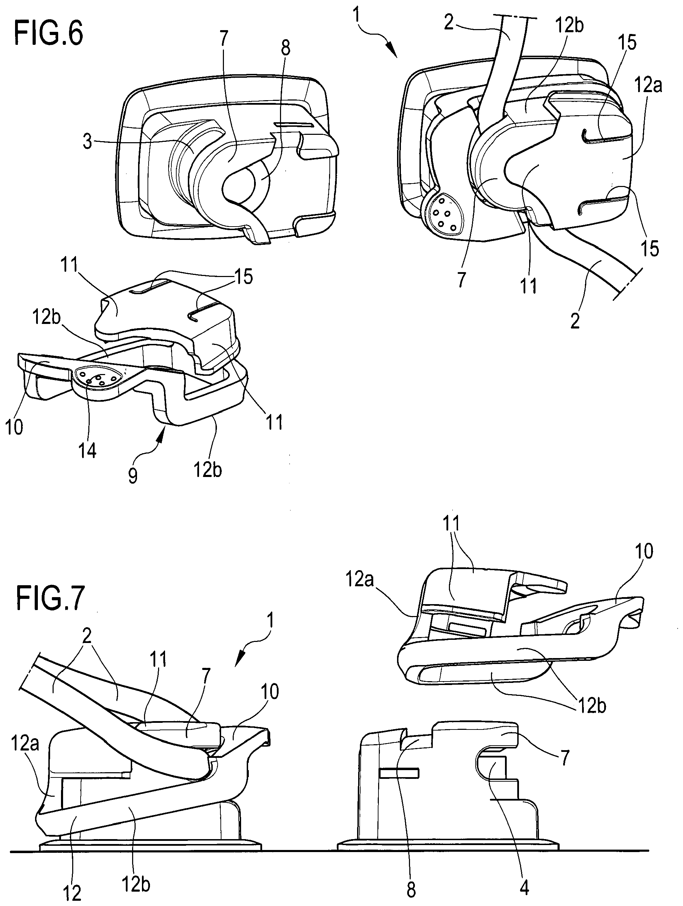

The head portion 7 is provided with the recessed housing 8 complementarity-shaped to at least a part of the mounting portion 11 of the holding element 9, such that the mounting portion 11 is stably insertable in the recessed seating 8, as is clearly visible in figure 6.

The head portion 7 of the device has an external surface, couplable with the mounting portion 11, which can be continuous and free of openings. The mounting portion 11 is conformed such as to maintain the holding element 9 stably mounted on the device in the engaged position and the access position. The retaining portion 10 comprises an activating portion 14 destined to enable and facilitate a manual displacement of the retaining portion 10. The mounting portion 11 exhibits an internal surface, couplable with the device 1, which can be free of projections insertable in the device.

The mounting portion 11 comprises at least two, at least three and in particular at least four rest surfaces which are transversal to one another and reciprocally connected and destined to enter into contact, when the holding element 9 is mounted on the clamping device 1, with corresponding surfaces connected to one another of the corresponding head portion 7 of the clamping device, in order to maintain the holding element 9 in position on the clamping device 1.

In particular, a first rest surface defines an upper zone of the mounting portion that is substantially flat.

As can be seen in figure 4, the first surface is slightly curved: the term "substantially flat" should be taken to mean a surface which is not necessary lying in a geometric plane but which has a mean flat development

The second, the third and the fourth rest surfaces define three lateral walls, consecutively arranged and emerging transversally from the first rest surface, destined to define a continuous lateral wall on three consecutive adjacent sides of the upper surface. The continuity is exclusively interrupted by two through-recesses 15 which interest a majority of the bottom lateral wall which is in turn connected to the support portion 12.

The cooperation between the four above-mentioned rest surfaces defines an internal cavity of the mounting portion 11 which is closed in the upper zone and on three sides (the three lateral walls).

Optionally, at least one and in particular two of the lateral walls (in particular opposite and facing) exhibit means for snap-fitting to a head portion of the device for clamping flexible hoses.

The snap-fitting means are defined, for example, by protuberances emerging projectingly from the respective lateral wall such as to engage corresponding seatings on the head portion (see figure 7); as an obvious alternative the protuberances could be on the head portion and the seatings on the walls, or also in any configuration that is a combination of the foregoing.

Note that the retaining portion 10 exhibits an activating surface defining a substantially flat upper zone which is also substantially parallel to the first rest surface of the mounting portion.

In both the access position and the engaged position, the retaining portion 10 is exclusively supported by the support portion 11 and in particular the retaining portion 10, in both the access position and the engaged position, is deconstrained from elements other than the support portion 11.

In other terms, the retaining portion 10 is defined by a continuous continuous tract having an upper surface that is larger than a vertical dimension thereof, in order to facilitate activation.

The continuous tract of the retaining portion connects and conjoins two respective ends of the support portion 12 by which it is exclusively supported.

The retaining portion 10 is in fact separated and in particular not in contact with the mounting surface (and optionally it is also not in contact with the head portion fo the clamping device), as it is maintained elastically suspended in both its operating positions (access/engagement) by the support portion 12.

Note also that the support portion 12 emerges inferiorly from the mounting portion 11 and develops below the mounting portion 11, re-emerging in an upwards direction such as to connect to the retaining portion 10; the movement from the engaged position to the access position occurs by means of a distancing movement of the retaining portion 10 from the plane containing the upper zone of the mounting portion 11 by means of at least a partial rotation about the support portion 12.

In other terms, the support portion 12 deforms elastically and enables the distancing of the retaining portion 10 from the mounting portion via a roto-translating motion which increases the distance between the portions 10 and 11 such as to create a sufficient gap which can enable an insertion of a tract of hose into the gap.

The retaining portion 10, the support portion 12 and the mounting portion 11 together define an internal seating destined to receive a head of a device for clamping hoses; the mountain portion 11 defines an upper rest surface for the head, while the support portion 12 surrounds the head on at least three contiguous sides; the retaining portion 10 is located at a fourth side of the head.

In this way the holding element 9 can be keyed on the head portion 7 until the upper rest surface abuts on the head portion; the snap-fitting means present on the lateral walls of the holding element 9 and on the head portion 7 couple and improve the reciprocal engagement; and the support and retaining portions 21, 10 surround the head portion and position such as to cooperate with the head portion 7, in use, for retaining and clamping the portion of hose.

In assembly conditions of the holding element to the head 7, the head 7 abuts on the mounting portion 11 and the support portion 12 comprises at least a pair of secondary arms 12b which embrace the head 7 on at least two sides thereof.

The retaining portion 10 joins the two respective ends of the secondary arms 12b and faces the head 7 at a further side thereof.

The holding element is realised in a single part.

In a variant which is not illustrated, the body might be realised in several reciprocally mounted parts. The holding element 9 can be of the single-use type, to enable a rapid replacement thereof with a like element which is whole and completely sterile. The body can be made of a plastic material, or another material suitable for the purpose. The invention further relates in a single aspect to a medical machine (not illustrated in the figures as of known type) comprising at least a clamping device 1 for flexible hoses 2 having at least a holding element 9 in accordance with what is described herein, and for example two clamping devices 1 and at least two corresponding holding elements 9 of the invention (illustrated for example in figures 1-2 and 5-7).

A device 1 and a holding element 9 of the present invention are applicable, for example, to a hemo(dia)filtration apparatus, in itself of known type and thus described only summarily in the following, comprising for example a hemo(dia)filter having a blood chamber and a fluid chamber that are separated from one another by a semipermeable membrane. The hemo(dia)filtration apparatus can further comprise a blood circuit connected to an inlet of the blood chamber, and a blood return line connected to an outlet of the blood chamber. The removal line is coupled to a blood pump for control of the extracorporeal circulation. The hemo(dia)filtration apparatus can further comprise a replacement fluid circuit having a pre-dilution line connected to the removal line and a post-dilution line connected to the return line. The replacement fluid circuit is provided with a replacement fluid circulation device comprising, for example, a positive displacement pump coupled to a main replacement fluid supply line which forks into the pre-dilution and post-dilution lines at a forking point arranged downstream of the pump.

The hemo(dia)filtration apparatus can further comprise a used fluid discharge line connected to an outlet of the fluid chamber and, in the case of hemo(dia)filtration, further comprises a dialysis fluid supply line connected to an inlet of the fluid chamber.

Each line is provided with a device for fluid circulation constituted for example by a positive displacement pump. The main replacement fluid supply line can be connected in a branched relation to the dialysis fluid supply line.

The hemo(dia)filtration apparatus is provided with the clamping device 1 for flexible hoses 2 as described herein above with reference to the accompanying figures of the drawings. In particular the illustrated tract of flexible hose 2 (associated to the pinch) can be a part, for example, of the removal line, the return line, the pre-dilution line or the post-dilution line. The clamping device 1 is connected to a control unit (not illustrated) of the hemo(dia)filtration apparatus and is controlled automatically by the unit. Alternatively an operator might manually activate the clamping device 1. Thanks to the clamping device 1 it is possible to selectively interrupt the blood circulation in the removal and/or return lines, for example in order to block circulation in the case of problems in the medical machine, or also control the flow distribution of the replacement fluid coming from the supply line and which is selectively directed to the pre-dilution or post-dilution lines by appropriate actuation of the clamping device 1.

The clamping device 1 of the accompanying figures further comprises a position sensor (for example a discrete position sensor, a Hall effect sensor, etc.) configured such as to emit at least a position signal which indicates at least a position of the piston; the position signal is received by a control unit (for example the control unit of the hemo(dia)filtration apparatus which, according to the position signal, controls the position of the piston of the device. The invention enables important advantages to be obtained and to obviate some of the drawbacks in the prior art.

A holding element, for clamping devices of flexible hoses in medical machine and a corresponding clamping device of the present invention can be easily and rapidly cleaned or disinfected in cases of necessity.

In particular a holding element of the invention is completely removable in order to facilitate the cleaning operations of the device and is replaceable in a case of breakage or for enabling rapid repositioning of a clean or sterile holding element on the relative device in order to perform a fresh treatment.

Further, a holding element and a corresponding device of the invention offer a high safety level against any possible contamination by the corporeal fluids passing through the flexible hoses. A holding element and a corresponding clamping device are reliable, simple, economical and easy to use.

## Claims

1. A clamping device for flexible hoses (2) in medical apparatus and a holding element (9) for flexible hose clamping device in medical apparatus,
wherein the device comprises:
- a housing seating (3) for receiving a tract of flexible hose (2);
- a pinch for flexible hoses (2) exhibiting a piston which is mobile in an axial direction, solidly bearing a pinching projecting element (4) cooperating with a fixed element (5) for abutting the seating (3) for occluding, by pinching, the tract of flexible hose (2) housed in the housing seating (3), the pinch being able to assume at least an open configuration in which the seating (3) receives the tract of flexible hose and at least a closed configuration in which it occludes, by pinching, the tract of hose received in the seating (3),
- wherein the holding element (9) has at least a body comprising:
- at least a mounting portion (11), removably mountable on the device (1) for clamping flexible hoses (2),
- at least a retaining portion (10) for the tract of a flexible hose (2) and
- at least a support portion (12), interposed between the mounting portion (11) and the retaining portion (10), the body being provided with at least an elastically deformable zone (13) for enabling selective displacement of the retaining portion (10) with respect to the mounting portion (11), when the holding element (9) is mounted on the device (1) for clamping flexible hoses (2), between an engaged position in which it is destined to maintain the tract of a flexible hose (2) in position with respect to the clamping device (1), and at least an access position, displaced with respect to the engaged position, in which it is destined to enable insertion and/or extraction of the tract of flexible hose (2) into and/or out of the clamping device (1), wherein the mounting portion (11) is conformed in such a way as to maintain the retaining portion (10) stably mounted on the device in the engaged position and in the access position of the retaining portion (10); the retaining portion (10) being destined, in the engaging position, to maintain the tract of flexible hose (2) in the housing seating (3) and being destined in the access position to allow insertion or extraction of said tract of flexible hose (2) into or from said housing seating (3).

2. The device and the element of claim 1, wherein the support portion (12) further comprises a first arm (12a) extending in a distancing direction from the mounting portion (11) and a pair of secondary arms (12b) extending in a distancing direction from the first arm (12a) up to two ends of the retaining portion (10), the retaining portion (10) optionally conjoining the ends of the secondary arms (12b).

3. The device and element of any one of the preceding claims, wherein the retaining portion (10) is conformed such as to be in the retaining position with the support portion (12) at rest and to be displaceable into the access position by means of a pushing action, in a first direction, destined to overcome an elastic resistance of the elastically deformable zone (13), and wherein the elastically deformable zone (13) is defined by two through-recesses (15) realised in the mounting portion (11) and defining a weakened zone destined to facilitate bending of the support portion (12).

4. The device and element of claim 2, wherein the retaining portion (10) is conformed such as to be in the holding position with the support portion (12) at rest and to be displaceable into the access position via a pushing action on the mounting portion (11) in a first direction towards the clamping device (1), in order to overcome an elastic resistance of the elastically deformable zone (13); the mounting portion (11) being releasable from the clamping device (1) by means of a pushing action on the mounting portion in a second direction coinciding with the first direction but moving in an opposite sense thereto.

5. The device and element of any one of the previous claims, wherein the mounting portion (11) comprises at least four support surfaces transversal of one another and reciprocally connected and destined to come in contact, when the holding element (9) is assembled on a clamping device (1), with reciprocally-connected corresponding surfaces of a head portion (7) of the clamping device (1), in order to maintain the holding element (9) in position on the clamping device (1), in particular a first rest surface defining a substantially-flat upper zone of the mounting portion, the second, third and fourth rest surfaces defining consecutively-arranged lateral walls arranged emerging transversally from the first rest surface to define an internal cavity of the mounting portion, optionally one and in particular two of the lateral walls exhibiting snap-fitting means to a head portion of the clamping device (1) for flexible hoses (2).

6. The device and element of the preceding claim, wherein the retaining portion (10) exhibits an activating surface defining a substantially flat upper zone that is substantially parallel to the first rest surface of the mounting portion.

7. The device and element of any one of the preceding claims, wherein the retaining portion (10), in both the access position and the engaged position, is exclusively supported by the support portion (11), in particular the retaining portion (10), in both the access position and the engaged position, being deconstrained from elements other than the support portion (11).

8. The device and element of any one of the preceding claims, wherein the mounting portion (11) comprises an upper zone which is substantially flat and wherein the retaining portion (10) exhibits an activating surface defining an upper zone that is substantially flat and substantially parallel to the first rest surface of the mounting portion, the support portion (12) emerging inferiorly from the mounting portion (11) and developing below the mounting portion (11) and re-emerging in an upwards direction such as to connect to the retaining portion (10), the displacement from the retaining position to the access position occurring by a distancing movement of the retaining portion from the plane containing the upper zone of the mounting portion (11) by means of at least a partial rotation about the support portion (12).

9. The device and element of any one of the preceding claims, wherein the retaining portion (10), the support portion (12) and the mounting portion (11) define an internal seating destined to receive a head of the hose-clamping device, the mounting portion (11) defining a rest surface for the head, the support portion (12) surrounding the head on at least three sides, the retaining portion (10) being located at a fourth side of the head.

10. The device and element of claim 1, wherein the device comprises a head portion (7) to which the mounting portion (11) of the holding element (9) is removably mountable, the head portion (7) of the device being provided with at least a recessed seating (8) positioned superiorly of the housing seating (3) and at the fixed element (5) for abutting the seating and being shaped complementarily to at least a part of the mounting portion (11) of the holding element (9), such that the mounting portion (11) is stably insertable in the recessed seating (8) in order to prevent accidental extraction of the holding element (9) from the device.

11. The device and element of claim 10, wherein in assembly conditions of the holding element to the head (7), the head (7) abuts on the mounting portion (11) and the support portion (12) comprises at least a pair of secondary arms (12b) which embrace the head (7) on at least two sides thereof, the retaining portion (10) joining the two respective ends of the secondary arms (12b) and facing the head (7) at a further side thereof.

12. The device and element of any one of claims 10 and 11, wherein the housing seating (3) exhibits a C-profile in section and has an elongate shape along a development direction coinciding with the direction of the flexible hose to be received, the retaining portion (10) being positioned with respect to the seating (3), in an engaged condition of the holding element to the head (7), such as to prevent an extraction of the flexible hose (2) from the seating (3), occluding the outlet hole from the seating (3), a displacement of the retaining portion (10) towards the access position freeing the outlet hole of the seating (3) and enabling the flexible hose (2) to be removed.

13. The device and element of any one of claims 1, 10, 11 and 12, wherein the device further comprises at least a position sensor configured such as to emit a position signal indicating at least a position of the piston, in particular the device further comprising a control unit which receives the position signal and which controls the position of the piston on a basis of a signal received.

14. The device and element of any one of claims 1, 10, 11, 12 and 13, wherein the device further comprises a presence detector (6) of the flexible hose (2) internally of the housing seating (3), for example the detector being connected to a control unit.

## Patentansprüche

1. Klemmvorrichtung für Flexschläuche (2) in medizinischen Einrichtungen und Halteelement (9) für Flexschlauchklemmvorrichtungen in medizinischen Einrichtungen,
wobei die Vorrichtung umfasst:
- einen Aufnahmesitz (3) zur Aufnahme eines Abschnitts des Flexschlauches (2);
- eine Klemme für Flexschläuche (2) aufweisend einen Kolben, der in einer Axialrichtung beweglich ist und der einstückig ein abstehendes Klemmelement (4) trägt, das mit einem feststehenden Element (5) zusammenwirkt zum Anschlagen an den Sitz (3) zum klemmenden Verschließen des Flexschlauchabschnitts (2), der im Aufnahmesitz (3) aufgenommen ist, wobei die Klemme mindestens eine geöffnete Konfiguration, bei der der Sitz (3) den Flexschlauchabschnitt aufnimmt, und mindestens eine geschlossene Konfiguration, bei der sie den im Sitz (3) aufgenommenen Flexschlauchabschnitt klemmend verschließt, annehmen kann,
- wobei das Halteelement (9) mindestens einen Körper besitzt, umfassend:
- mindestens einen Montagebereich (11), der an der Klemmvorrichtung (1) für Schläuche (2) lösbar montierbar ist,
- mindestens einen Fixierbereich (10) für den Flexschlauchabschnitt (2), und
- mindestens einen Tragbereich (12), der zwischen dem Montagebereich (11) und dem Fixierbereich (10) liegt, wobei der Körper mit mindestens einer elastisch verformbaren Zone (13) versehen ist, zum Ermöglichen der selektiven Verschiebung des Fixierbereichs (10) bezüglich des Montagebereichs (11), wenn das Halteelement (9) an der Klemmvorrichtung (1) für Flexschläuche (2) montiert ist, zwischen einer Eingriffsstellung, bei der es zum Halten eines Flexschlauchabschnitts (2) bezüglich der Klemmvorrichtung (1) vorgesehen ist, und mindestens einer Zugangsstellung, die bezüglich der Eingriffsstellung verschoben ist, bei der es zum Ermöglichen der Einführung und/oder Entnahme des Flexschlauchabschnitts (2) in die/aus der Klemmvorrichtung (1) vorgesehen ist, wobei der Montagebereich (11) so ausgestaltet ist, den Fixierbereich (10) an der Vorrichtung in der Eingriffsstellung und in der Zugangsstellung des Fixierbereichs (10) fest montiert zu halten;
wobei der Fixierbereich (10) in der Eingriffsstellung vorgesehen ist, den Flexschlauchabschnitt (2) im Aufnahmesitz (3) zu halten, und in der Zugangsstellung vorgesehen ist, die Einführung oder Entnahme des Flexschlauchabschnitts (2) in den oder aus dem Aufnahmesitz (3) zu ermöglichen.

2. Vorrichtung und Element nach Anspruch 1, wobei der Tragbereich (12) weiter einen ersten Arm (12a), der sich in einer sich absetzenden Richtung vom Montagebereich (11) erstreckt, und ein Paar von zweiten Armen (12b), die sich in einer sich absetzenden Richtung vom ersten Arm (12a) bis hin zu zwei Enden des Fixierbereichs (10) erstrecken, umfasst, wobei der Fixierbereich (10) optional die Enden der zweiten Armen (12b) verbindet.

3. Vorrichtung und Element nach einem der vorhergehenden Ansprüche, wobei der Fixierbereich (10) so ausgestaltet ist, in der Fixierstellung mit dem Tragbereich (12) aufliegend zu sein und in die Zugangsstellung verschiebbar zu sein, durch eine Schubwirkung in einer ersten Richtung, die zur Überwindung eines elastischen Widerstands der elastisch verformbaren Zone (13) vorgesehen ist, und wobei die elastisch verformbare Zone (13) durch zwei durchgehende Ausnehmungen (15) definiert ist, die im Montagebereich (11) ausgebildet sind und eine abgeschwächte Zone definieren, die zum Erleichtern der Biegung des Tragbereichs (12) vorgesehen ist.

4. Vorrichtung und Element nach Anspruch 2, wobei der Fixierbereich (10) so ausgestaltet ist, in der Haltestellung mit dem Tragbereich (12) aufliegend zu sein und in die Zugangsstellung verschiebbar zu sein, durch eine Schubwirkung am Montagebereich (11) in einer ersten Richtung zur Klemmvorrichtung (1), um einen elastischen Widerstand der elastisch verformbaren Zone (13) zu überwinden; wobei der Montagebereich (11) von der Klemmvorrichtung (1) lösbar ist, durch eine Schubwirkung am Montagebereich in einer zweiten Richtung, die der ersten Richtung entspricht aber sich in einem dazu entgegengesetzten Sinn bewegt.

5. Vorrichtung und Element nach einem der vorhergehenden Ansprüche, wobei der Montagebereich (11) mindesten vier zueinander querliegende, miteinander verbundene Tragflächen umfasst, die in Kontakt zu kommen vorgesehen sind, wenn das Halteelement (9) an einer Klemmvorrichtung (1) montiert wird, mit miteinander verbundenen, entsprechenden Flächen eines Kopfbereichs (7) der Klemmvorrichtung (1), um das Halteelement (9) an der Klemmvorrichtung (1) zu halten, wobei insbesondere eine erste Auflagefläche eine im Wesentlichen flache obere Zone des Montagebereichs definiert, wobei die zweite, dritte und vierte Auflageflächen aufeinanderfolgende Seitenwände definieren, die von der ersten Auflagefläche quer abstehend angeordnet sind, um einen inneren Hohlraum des Montagebereichs zu definieren, wobei optional eine oder insbesondere zwei der Seitenwände Steckmittel zur Verbindung mit einem Kopfbereich der Klemmvorrichtung (1) für Schläuche (2) besitzen.

6. Vorrichtung und Element nach dem vorhergehenden Anspruch, wobei der Fixierbereich (10) eine Aktivierungsfläche besitzt, die eine im Wesentlichen flache obere Zone definiert, die im Wesentlichen parallel zur ersten Auflagefläche des Montagebereichs ist.

7. Vorrichtung und Element nach einem der vorhergehenden Ansprüche, wobei der Fixierbereich (10), sowohl in der Zugangsstellung als auch in der Eingriffsstellung, nur durch den Tragbereich (11) getragen wird, wobei insbesondere der Fixierbereich (10), sowohl in der Zugangsstellung als auch in der Eingriffsstellung, von anderen Elementen als dem Tragbereich (11) gelöst ist.

8. Vorrichtung und Element nach einem der vorhergehenden Ansprüche, wobei der Montagebereich (11) eine im Wesentlichen flache obere Zone umfasst, und wobei der Fixierbereich (10) eine Aktivierungsfläche aufweist, die eine obere Zone definiert, die im Wesentlichen flach und im Wesentlichen parallel zur ersten Auflagefläche des Montagebereichs ist, wobei der Tragbereich (12) unterhalb vom Montagebereich (11) absteht und sich unter des Montagebereich (11) entwickelt und in einer oben gerichteten Richtung wieder absteht, um sich mit dem Fixierbereich (10) zu verbinden, wobei die Verschiebung von der Fixierstellung zur Zugangsstellung durch eine Entfernungsbewegung des Fixierbereichs von der Ebene, die die obere Zone des Montagebereichs (11) enthält, mittels mindestens einer Teildrehung um den Tragbereich (12) stattfindet.

9. Vorrichtung und Element nach einem der vorhergehenden Ansprüche, wobei der Fixierbereich (10), der Tragbereich (12) und der Montagebereich (11) einen Innensitz definieren zur Aufnahme eines Kopfs der Flexschlauchklemmvorrichtung, wobei der Montagebereich (11) eine Auflagefläche für den Kopf definiert, wobei der Tragbereich (12) den Kopf an mindestens drei Seiten umgibt, wobei der Fixierbereich (10) an einer vierten Seite des Kopfs liegt.

10. Vorrichtung und Element nach Anspruch 1, wobei die Vorrichtung einen Kopfbereich (7) umfasst, an dem der Montagebereich (11) des Halteelements (9) lösbar montierbar ist, wobei der Kopfbereich (7) der Vorrichtung mit mindestens einem versenkten Sitz (8) versehen ist, der über dem Aufnahmesitz (3) und am feststehenden Element (5) zum Aufliegen am Sitz positioniert ist, und komplementär zu mindestens einem Teil des Montagebereichs (11) des Halteelements (9) ausgebildet ist, so dass der Montagebereich (11) in den versenkten Sitz (8) fest einführbar ist, um eine ungewollte Entnahme des Halteelements (9) aus der Vorrichtung zu verhindern.

11. Vorrichtung und Element nach Anspruch 10, wobei in einer Montagelage des Halteelements am Kopf (7) der Kopf (7) am Montagebereich (11) anliegt und der Tragbereich (12) mindestens ein Paar von zweiten Armen (12b), die den Kopf (7) an mindestens zwei Seiten davon umschließen, umfasst, wobei der Fixierbereich (10) die beiden jeweiligen Enden der zweiten Arme (12b) verbindet und dem Kopf (7) an einer weiteren Seite davon gegenüberliegt.

12. Vorrichtung und Element nach einem der Ansprüche 10 und 11, wobei der Aufnahmesitz (3) in Querschnitt ein C-förmiges Profil aufweist und eine längliche Form besitzt, entlang einer Entwicklungsrichtung entsprechend der Richtung des aufzunehmenden Flexschlauchs, wobei der Fixierbereich (10) bezüglich dem Sitz (3) positioniert ist, in einer eingegriffenen Lage des Halteelements am Kopf (7), um eine Entnahme des Flexschlauchs (2) aus dem Sitz (3) zu verhindern, was das Auslassloch des Sitzes (3) verschließt, wobei eine Verschiebung des Fixierbereichs (10) zur Zugangsstellung das Auslassloch des Sitzes (3) frei macht und die Entnahme des Flexschlauchs (2) ermöglicht.

13. Vorrichtung und Element nach einem der Ansprüche 1, 10, 11 und 12, wobei die Vorrichtung weiter mindestens einen Positionssensor umfasst, der konfiguriert ist zum Aussenden eines Positionssignals, das mindestens eine Position des Sensors angibt, wobei insbesondere die Vorrichtung weiter eine Steuereinheit umfasst, die das Positionssignal empfängt und die Position des Kolbens basierend auf einem empfangenen Signal steuert.

14. Vorrichtung und Element nach einem der Ansprüche 1, 10, 11, 12 und 13, wobei die Vorrichtung weiter einen Anwesenheitsdetektor (6) des Flexschlauchs (2) im Aufnahmesitz (3) umfasst, wobei zum Beispiel der Detektor mit einer Steuereinheit verbunden ist.

## Revendications

1. Dispositif de serrage de tubes souples (2) dans des appareils médicaux et élément de maintien (9) pour dispositifs de serrage de tubes souples dans des appareils médicaux,
où le dispositif comprend :
- un siège de logement (3) apte à recevoir une partie de tube souple (2) ;
- une pince pour tubes souples (2) présentant un piston qui est mobile dans une direction axiale, supportant intégralement un élément saillant de serrage (4) coopérant avec un élément fixe (5) pour s'attester contre le siège (3) pour fermer en pinçant la partie de tube souple (2) logée dans le siège de logement (3), la pince étant à même de prendre au moins une configuration ouverte dans laquelle le siège (3) reçoit la partie de tube souple et au moins une configuration fermée dans laquelle elle ferme en pinçant la partie de tube reçue dans le siège (3),
- où l'élément de maintien (9) a au moins un corps comprenant :
- au moins une portion de montage (11) pouvant être montée de façon amovible sur le dispositif (1) pour serrer des tubes souples (2),
- au moins une portion de retenue (10) pour la partie d'un tube souple (2), e
- au moins une portion de support (12), placée entre la portion de montage (11) et la portion de retenue (10), le corps étant muni d'au moins une zone déformable élastiquement (13) pour permettre le déplacement sélectif de la portion de retenue (10) par rapport à la portion de montage (11), lorsque l'élément de maintien (9) est monté sur le dispositif (1) pour serrer des tubes souples (2) entre une position engagée dans laquelle elle est destinée à maintenir la partie d'un tube souple (2) en position par rapport au dispositif de serrage (1), et au moins une position d'accès, décalée par rapport à la position engagée, dans laquelle elle est destinée à permettre l'insertion et/ou l'extraction de la partie de tube souple (2) dans le et/ou du dispositif de serrage (1), où la portion de montage (11) est conformée de sorte à maintenir la portion de retenue (10) montée stabile sur le dispositif dans la position engagée et dans la position d'accès de la portion de retenue (10) ;
la portion de retenue (10) étant destinée, dans la position engagée, à maintenir la partie de tube souple (2) dans le siège de logement (3) et étant destinée, dans la position d'accès, à permettre l'insertion ou l'extraction de ladite partie de tube souple (2) dans ledit ou dudit siège de logement (3).

2. Dispositif et élément selon la revendication 1, où la portion de support (12) comprend en outre un premier bras (12a) s'étendant dans une direction d'éloignement de la portion de montage (11) et une paire de bras secondaires (12b) s'étendant dans une direction d'éloignement du premier bras (12a) jusqu'à deux extrémités de la portion de retenue (10), la portion de retenue (10) reliant optionnellement les extrémités des bras secondaires (12b).

3. Dispositif et élément selon l'une quelconque des revendications précédentes, où la portion de retenue (10) est conformée de sorte à être dans la position de retenue avec la portion de support (12) en appui et à être déplaçable dans la position d'accès par l'intermédiaire d'une action de poussée, dans une première direction, destinée à surpasser une résistance élastique de la zone élastiquement déformable (13), et où la zone élastiquement déformable (13) est définie par deux niches passantes (15) réalisées dans la portion de montage (11) et définissant une zone affaiblie destinée à faciliter la flexion de la portion de support (12).

4. Dispositif et élément selon la revendication 2, où la portion de retenue (10) est conformée de sorte à être dans la position de maintien avec la portion de support (12) en appui et à être déplaçable dans la position d'accès par l'intermédiaire d'une action de poussée sur la portion de montage (11) dans une première direction vers le dispositif de serrage (1), afin de surpasser une résistance élastique de la zone élastiquement déformable (13) ; la portion de montage (11) pouvant être dégagée du dispositif de serrage (1) par l'intermédiaire d'une action de poussée sur la portion de montage dans une deuxième direction correspondant à la première direction mais se déplaçant dans un sens opposé à celle-ci.

5. Dispositif et élément selon l'une quelconque des revendications précédentes, où la portion de montage (11) comprend au moins quatre surfaces de support les unes transversales aux autres et réciproquement liées et destinées à venir en contact, lorsque l'élément de maintien (9) est monté sur un dispositif de serrage (1), avec des surfaces correspondantes réciproquement liées d'une portion de tête (7) du dispositif de serrage (1), afin de maintenir l'élément de maintien (9) en position sur le dispositif de serrage (1), en particulier une première surface d'appui définissant une zone supérieure sensiblement plate de la portion de montage, les deuxième, troisième et quatrièmes surfaces d'appui définissant des parois latérales rangées de façon consécutive et saillant transversalement de la première surface d'appui pour définir une cavité intérieure de la portion de montage, optionnellement une et en particulier deux des parois latérales présentant des moyens de raccordement à pression à une portion de tête du dispositif de serrage (1) pour tubes souples (2).

6. Dispositif et élément selon la revendication précédente, où la portion de retenue (10) présente une surface d'activation définissant une zone supérieure sensiblement plate qui est sensiblement parallèle à la première surface d'appui de la portion de montage.

7. Dispositif et élément selon l'une quelconque des revendications précédentes, où la portion de retenue (10), tant dans la position d'accès que dans la position engagée, est supportée seulement par la portion de support (11), en particulier la portion de retenue (10), tant dans la position d'accès que dans la position engagée, étant détachée d'autres éléments que la portion de support (11).

8. Dispositif et élément selon l'une quelconque des revendications précédentes, où la portion de montage (11) comprend une zone supérieure qui est sensiblement plate, et où la portion de retenue (10) présente une surface d'activation définissant une zone supérieure qui est sensiblement plate et sensiblement parallèle à la première surface d'appui de la portion de montage, la portion de support (12) saillant de dessous de la portion de montage (11) et se développant au-dessous de la portion de montage (11) et saillant dans une direction vers le haut de sorte à se relier à la portion de retenue (10), le déplacement de la position de retenue à la position d'accès ayant lieu par un mouvement d'éloignement de la portion de retenue du plan contenant la zone supérieure de la portion de montage (11) par l'intermédiaire d'au moins une rotation partielle autour de la portion de support (12).

9. Dispositif et élément selon l'une quelconque des revendications précédentes, où la portion de retenue (10), la portion de support (12) et la portion de montage (11) définissent un siège intérieur destiné à recevoir une tête du dispositif de serrage de tubes souples, la portion de montage (11) définissant une surface d'appui pour la tête, la portion de support (12) entourant la tête sur au moins trois côtés, la portion de retenue (10) étant placée sur un quatrième côté de la tête.

10. Dispositif et élément selon la revendication 1, où le dispositif comprend une portion de tête (7) à laquelle la portion de montage (11) de l'élément de maintien (9) peut être montée de façon amovible, la portion de tête (7) du dispositif étant munie d'au moins un siège retrait (8) positionné au-dessus du siège de logement (3) et sur l'élément fixe (5) pour s'attester contre le siège et étant conformée de façon complémentaire à au moins une partie de la portion de montage (11) de l'élément de maintien (9), de sorte que la portion de montage (11) peut être insérée de façon stabile dans le siège retrait (8) afin d'empêcher l'extraction accidentelle de l'élément de maintien (9) du dispositif.

11. Dispositif et élément selon la revendication 10, où dans des conditions d'assemblage de l'élément de maintien à la tête (7), la tête (7) s'atteste contre la portion de montage (11) et la portion de support (12) comprend au moins une paire de bras secondaires (12b) qui entourent la tête (7) sur au moins deux côtés de celle-ci, la portion de retenue (10) reliant les deux extrémités respectives des bras secondaires (12b) et se trouvant en face de la tête (7) sur un autre côté de celle-ci.

12. Dispositif et élément selon l'une quelconque des revendications 10 et 11, où le siège de logement (3) présente un profil en C en section et a une forme allongée le long d'une direction de développement correspondant à la direction du tube souple à recevoir, la portion de retenue (10) étant positionnée par rapport au siège (3), dans une condition engagée de l'élément de maintien à la tête (7), de sorte à empêcher une extraction du tube souple (2) du siège (3), fermant le trou de sortie du siège (3), un déplacement de la portion de retenue (10) vers la position d'accès dégageant le trou d'accès du siège (3) et permettant l'enlèvement du tube souple (2).

13. Dispositif et élément selon l'une quelconque des revendications 1, 10, 11 et 12, où le dispositif comprend en outre au moins un capteur de position configuré de sorte à émettre un signal de position indiquant au moins une position du piston, en particulier le dispositif comprenant en outre une unité de commande qui reçoit le signal de position et commande la position du piston sur la base du signal reçu.

14. Dispositif et élément selon l'une quelconque des revendications 1, 10, 11, 12 et 13, où le dispositif comprend en outre un détecteur de présence (6) du tube souple (2) à l'intérieur du siège de logement (3), par exemple le détecteur étant relié à une unité de commande.
